# EUROPEAN PATENT SPECIFICATION

(11) **EP 1 417 983 B1**
(45) Date of publication and mention of the grant of the patent: **14.06.2006**
(21) Application number: 03024842.1
(22) Date of filing: 31.10.2003
(51) Int. Cl.: A61M 25/06

(54) **Cannula needle or catheter needle with safety device**
Kanüle-Nadel oder Katheterführungsnadel mit Sicherheitseinrichtung
Aiguille-canule ou aiguille-catheter avec un dispositif de sécurité

(30) Priority: 05.11.2002 IT FR20020033
(43) Date of publication of application: 12.05.2004
(73) Proprietor: ARTSANA S.p.A., 22070 Grandate (Como) (IT)
(72) Inventor: Mattoni, Guglielmo, 03020 Castro dei Volsci (Frosinone) (IT)
(74) Representative: Ripamonti, Enrico

(56) References cited:
- EP-A- 0 499 077
- EP-A- 0 875 261
- US-A- 5 171 231
- US-A- 5 456 668
- US-A- 5 695 476
- US-A- 5 954 698
- US-A- 6 050 976

## Description

The present invention relates to a cannula needle or catheter needle in accordance with the introduction to the main claim.

A cannula needle of the aforedescribed type comprises a first and a second portion which are mutually separable. The first portion comprises a catheter (usually of biocompatible plastic material) to be inserted into a patient's vein, for example of the arm, and secured to a carrier to be fixed (in known manner) to the patient, for example the patient's arm, in proximity to said vein. The second portion comprises a metal needle which, to stiffen it, is inserted into the cannula on inserting this latter into the vein. The needle also and in particular serves for cutting through the epidermis and penetrating into the vein, to enable the catheter tube to be inserted into the vein. The needle is associated with a support element. When the catheter and needle have been inserted into the vein, the needle is extracted from the catheter and from the vein and the second portion of the cannula needle is separated from the first. With this latter, or rather with the carrier carrying the catheter, any member, for example a tube, can then be associated to transfer a medical product from a vessel (for example a bag) to the vein.

Examples of cannula needle assemblies of the above mentioned kind are described in US 6050976, US 5456668, EP 0875261, US 5695476, US 5954698, US 5171231 and EP 499077. Generally the cannula needle assemblies of the prior art are not longitudinally compact when the metal needle is inserted into the catheter; hence their manipulation is not easy.

This is an important drawback in view of the fact that the catheter or cannula needle can be used in urgent intervention on a patient, that the longitudinal length of said cannula needle assembly may be able to negatively affect its operability.

US 6050979 describes a cannula or catheter needle assembly which is more compact than the other known solutions described in the above prior art. It describes an apparatus having particular actuators to detach the needle from the catheter and which exert a force on the needle to insert it into a safe containment.

The cannula needle can be one or two-way, depending on whether the first portion can be connected to one or two of said members for transferring a medical product into the vein.

During the aforedescribed separation of the needle of the second portion from the body of the first portion it can happen that the medical operator or nurse assisting the patient can be accidentally pricked with the needle, with possible contraction of serious and dangerous infections.

An object of the present invention is to eliminate said risk of contact with the needle, to hence ensure safety of the operator assisting the patient. A particular object of the invention is to provide a cannula needle or catheter needle which enables this aim to be achieved, so adequately protecting the operator.

Another object is to provide a cannula needle of the stated type which is of simple construction and use, is of small overall size, and can be used without negatively affecting the operability of the cannula needle.

These and other objects which will be apparent to the expert of the art are attained by a cannula needle or catheter needle in accordance with the accompanying claims.

The present invention will be more apparent from the accompanying drawing, which is provided by way of non-limiting example and in which:
Figure 1 is a perspective view from the rear, showing a catheter needle according to the invention;
Figure 2 is a right side view of one embodiment of the catheter needle of Figure 1 during a first phase of its use;
Figure 3 is a perspective view from the rear, showing the catheter needle of Figure 1 1 in a second phase of its use;

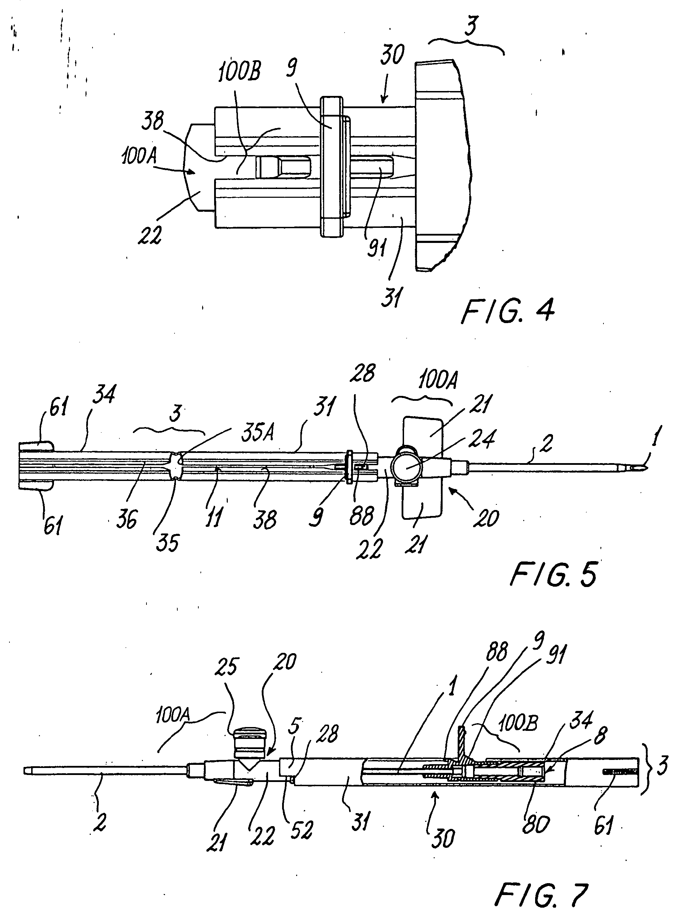

Figure 4 is a view from above of the part indicated by A in Figure 3, but seen from the right in the direction of the arrow K;
Figure 5 is a view of the catheter needle of Figure 1 from above in a third phase of its use;
Figure 6 is a perspective view of the catheter needle of Figure 5 from the rear;
Figure 7 is a partly sectional right side view of the catheter needle during a different phase of its use;
Figure 8 is a perspective view of the catheter needle of Figure 1 from the rear in a further phase of its use;
Figure 9 is a partly sectional side view of the catheter needle of Figure 8;

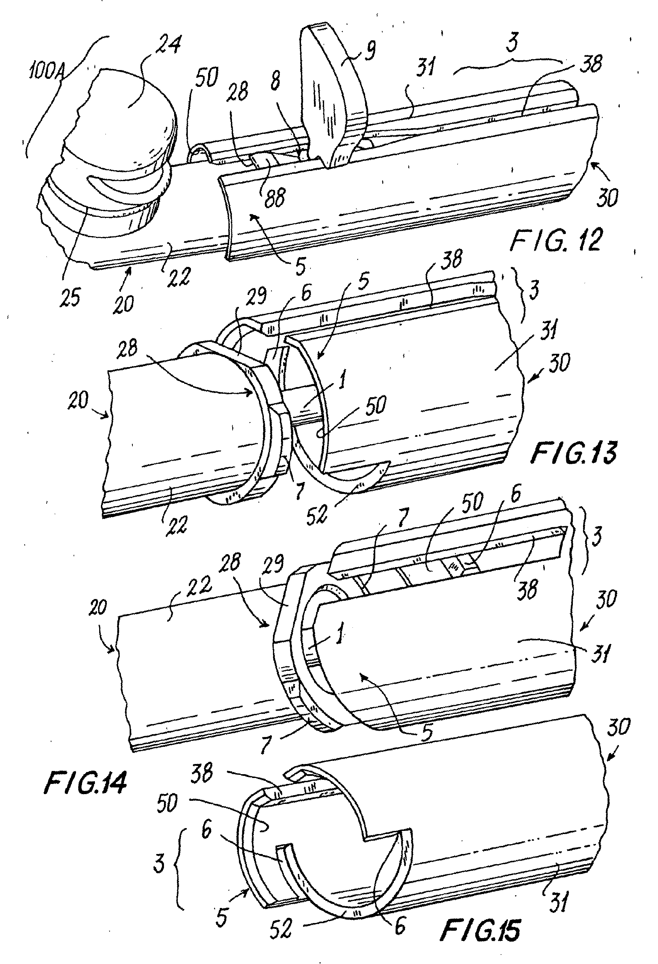

Figures 10 and 11 are enlarged views of the portions indicated respectively by B in Figure 9 and by C in Figure 10;
Figure 12 is an enlarged perspective view of the part indicated by A in Figure 1, seen in the direction of the arrow K shown therein;
Figure 13 is a partial enlarged exploded perspective view of parts of the cannula needle of Figure 1;
Figure 14 is an exploded view of Figure 13 from another viewpoint; and
Figure 15 is a partial enlarged perspective view of a protection member for the cannula needle of Figure 1.

With reference to said figures and to the means illustrated therein, a catheter needle 100 is shown presenting two portions 100A and 100B. The first portion 100A comprises a catheter tube 2 (or simply "catheter") associated with and carried by a carrier 20 presenting fins 21 to enable it to be secured to a part of the patient's body in proximity to a vein in which the catheter is to be inserted. The carrier 20 presents a hollow tubular end 22 positioned coaxial with the catheter 2 and connected to this latter, and a hollow tubular appendix 23 provided with a plug 24 on its end opening 25 and also connected to the catheter. The catheter 2 is of known biocompatible material.

The catheter needle 100 of the figures is of the two-way type, however the invention is-also applicable to a one-way catheter-lacking the tubular appendix 23. The second portion 100B comprises a metal needle 1 fixed to and carried by a support element 8. The needle 1 is positioned within the catheter 2 and is, inter alia, necessary for stiffening said catheter to enable it to penetrate into the vein. When the catheter 2 has penetrated therein, the metal needle 1 is extracted from it. Then in known manner, the needle 1 is separated from the carrier 20 which instead is fixed to the patient in proximity to the vein, for example of the arm, where the catheter 2 remains inserted.

The tubular end 22 and/or, if present, the appendix 23 are then connected, if required, to a tube for feeding a medical liquid to the patient, to thus connect the portion 100A of the catheter needle 100 to a vessel of this liquid.

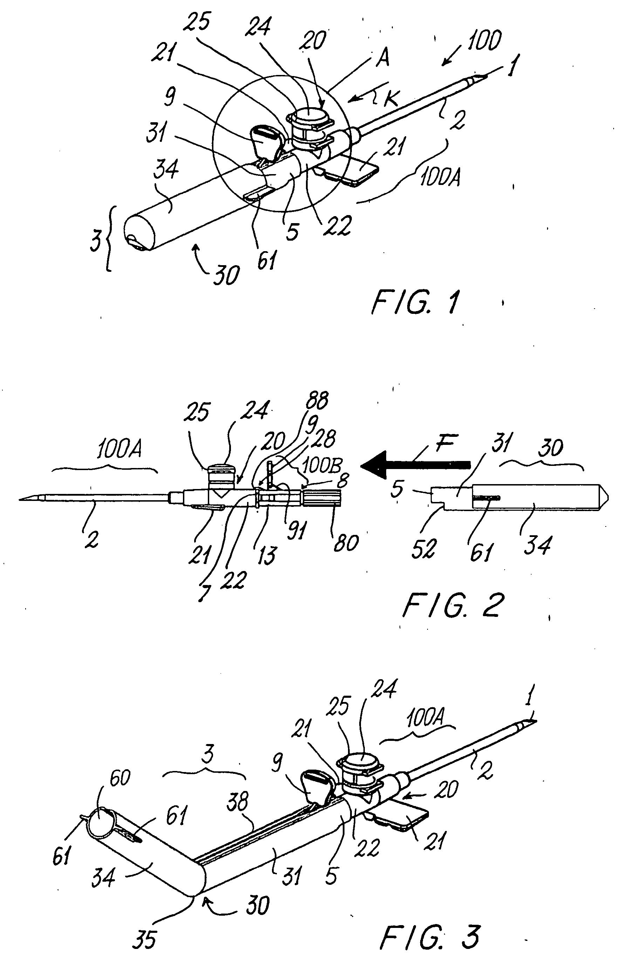

To prevent the metal needle 1 from being able to wound an operator (doctor or nurse), the needle is inserted into a portion 3 on being separated from the portion 100A of the cannula needle 100, This protection member 3 comprises a tubular element 30 preferably of plastic material presenting a portion 31 to be coupled (arrow F of Figure 2), via an elastically deformable end or connector 5, to the hollow tubular end 22 of the carrier 20 of the portion 100A. For this purpose, the end 5 comprises, within an inner wall 50, two semicircular and/or arched recesses or cuts 6 arranged to receive projecting portions 7 jutting, in opposing positions, from a flanged edge 28 at the hollow tubular end 22. This edge also presents at least one flattened portion 29 between the opposing portions 7. The insertion of these projecting portions 7 into the recesses 6 enables the tubular element 30 to be snap-coupled to the tubular end 22; likewise the flattened portion 29 is brought substantially to bear against the inner wall 50 of the end 5. This latter also presents an end recess 52 into which the recesses 6 in the wall 50 open, this end recess hence giving said end a "jutting" shape with respect to the portion 31 of the tubular element 30 of the protection member 3.

Preferably, the tubular element 30 comprises a second portion 34 movable relative to the first portion 31 and snap-closable onto this latter (see Figures 1, 2 and 3). In this respect, this second portion 34 is connected to the first via a weakening line 35 and a recess 35A similar to a V. The second portion also comprises a slot 36 which gives this portion 34 an open annular section shape such that it can deform elastically and overlay the first portion 31 to enable the tubular element 30 of the protection member 3 to have a longitudinally compact form and to facilitate its manipulation when it is coupled to the portion 100A of the catheter needle 100, while having however a size such as to receive the metal needle 1 when the portions 31 and 34 are adjacent.

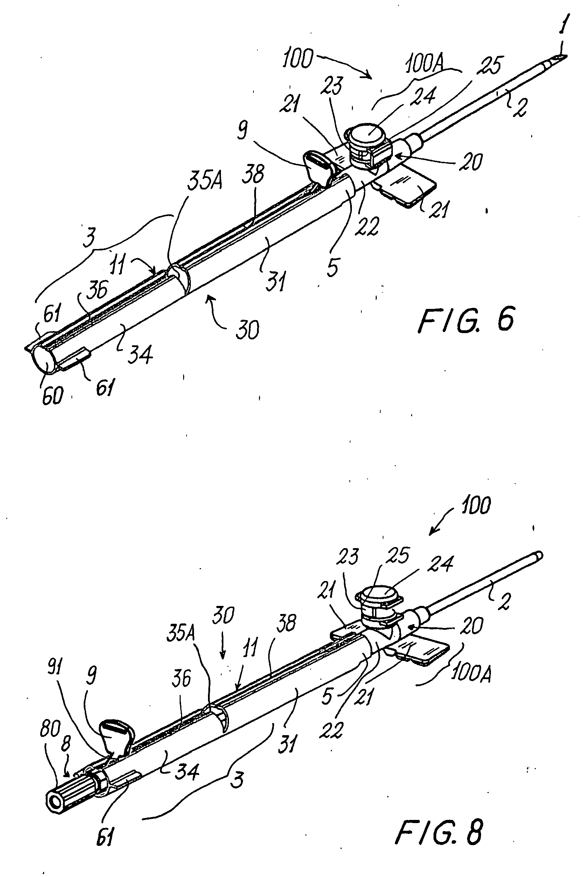

The slot 36 has a corresponding slot 38 provided in the first portion 31 which is essentially a continuation of the slot 36 and defines therewith a slot 11 which extends along the entire tubular body 30 of the protection member 3 when this is in its extended position (see for example Figures 5 and 6), i.e. it has its portions 31 and 34 adjacent.

The portion 34 is open at its free end 60 and in proximity to this latter presents projecting radial fins 61. These fins facilitate gripping of the portion 34 and its manipulation relative to the portion 31 when this is separated from the second.

An end portion or component 80 of the support element 8 of the metal needle 1 projects from (or within the tubular element 30 reaches at least in proximity to) the open end 60 when the needle 1 is separated from the portion 100A of the catheter needle 100. The needle 1 is movable by virtue of a gripping element or fin 9 rigid with said support element 8 via a possible rear stiffening mount 91. By acting on the fin 9 and by virtue of the slot 11 of the tubular element 30 within which the mount 91 can slide if provided, or if not then that portion of the fin 9 rigid with the support element 8, the metal needle 1 can be moved axially within the element 30 and can be made to retract into this latter when it is extracted from the catheter. This movement along the longitudinal axis of the element 30 terminates when a tooth or projection 14 provided in the interior of said element penetrates into a recess 13 (or an annular groove) provided in the support element 8.

The engagement between the tooth and said recess locks the support element 8 within the protection member 3 by virtue of the fact that the dimensions of said protection device (when in its extended form) are such that when this engagement between the tooth 14 and the recess 13 takes place, the metal needle 1 is inserted into the tubular element 30 and said needle can be safely separated from the portion 100A of the cannula needle 100 without risk of accidentally wounding the operator who has inserted the cannula 2 into the vein.

At this point, it is not possible to slide the projecting portions 7 of the flanged edge 28 of the tubular end 22 of said portion 100A towards the recess 52 within the semicircular recesses 6 of the tubular element if the metal needle 1 has not completely retracted into this element, because otherwise it would oppose the movement-of the end 22 towards said recess 52.

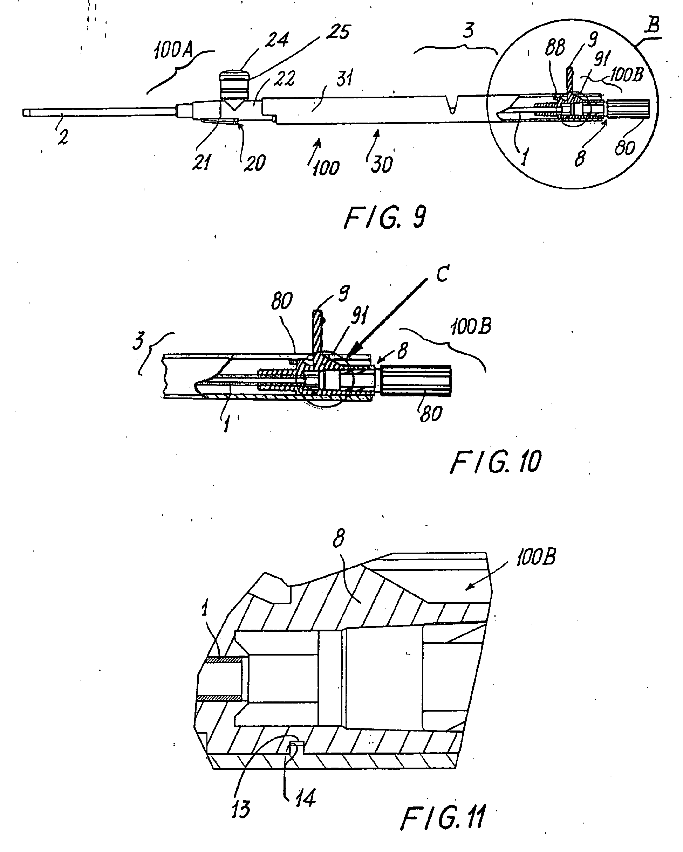

It should also be noted that the second portion 100B of the cannula needle 100 presents close to its free end 110 a projection 88 cooperating with the flanged edge 28 to define a limit stop for the connection between this portion 100B and the portion 100A as shown in Figures 7, 8 and 10. This projection projects both vertically from the portion 100B (and penetrates into the slot 11 of the protection device 3) and axially therefrom (i.e. it projects forward towards the portion 100A) in such a manner as to overlie the flattened portion 29 of said flanged edge and to rest against it to halt the insertion of the metal needle 1 into the catheter 2.

Finally, the whole of said portion 100B, or only its fin 9, is constructed of transparent material to enable the blood penetrating into it when the metal needle 1 and its catheter 2 are inserted into the vein to be seen. This function of the portion 100B or of its fin (highlighting function) is important in order to verify that the needle 1 and its catheter 2 are correctly positioned in the vein.

The invention offers considerable advantages compared with known cannula needles, mainly its safety during use at the moment in which the metal needle 1 is separated from the catheter 2, and safety in disposing of the used needle; in effect, in neither case can the needle accidentally wound any person because it is securely held within the protection device 3.

Finally, it should be noted that the protection device 3 can be already associated with the cannula needle 100 when sold, or can be considered an accessory separate from the cannula needle and connectable later thereto.

## Claims

1. A cannula needle or a catheter needle (100) comprising a first portion (100A) comprising a catheter (2) to be inserted into and maintained in a vein of a patient, and a second portion (100B) comprising a metal needle (1) inserted into the catheter (2) on inserting this latter into the vein and then being separated from it, said metal needle (1) being associated with a support element (8), said catheter (2) being secured to a carrier (20) to be fixed to the patient when said catheter (2) is inserted into the patient's vein, a tubular protection member (3) being associated with the carrier (20) of the first portion (100A) so as to contain therein the support element (8) for the metal needle (1) when this latter is still within the catheter (2), said tubular protection member (3) presenting a longitudinal aperture (11) within which guide means (9) for the metal needle (1) associated with the second portion (100B) of said cannula needle (100) can slide when the metal needle (1) is separated from the catheter (2), said tubular protection member (3) containing said metal needle (1) when the second portion (100B) of the cannula needle (100) is separated from the first (100A) and the catheter (2) is maintained within the vein, **characterised in that** the tubular protection member (3) comprises a plurality of foldable portions (31, 34) arranged to be closable one over the other when said member (3) is associated with the carrier (20) and the metal needle (1) is inserted into the catheter (2) so as to reduce the protection member length, said portions (31, 34) being separated and being put adjacent so as to receive the metal needle (1) when it is detached from said first portion (100A) of the cannula needle (100), the protection member (3) being defined by a tubular element (30) comprising two hinged together superposable portions (31, 34), the first (31) of these portions (31, 34) being able to be removably coupled to the carrier (20) to which the catheter (2) is secured.

2. A cannula needle as claimed in claim 1, **characterised by** comprising, the needle second portion (100B) has locking means associated with said tubular protection member (3) to lock the movement of said second portion (100B) when the metal needle (1), separated from the catheter (2), is completely contained within the tubular protection member (3) so as to be protected by this latter when said second needle portion (100B) is separated from the first needle portion (100A) with said tubular protection member (3).

3. A cannula needle as claimed in claim 1, **characterised in that** said first portion (31) of the tubular element (30) defining the protection member (3) comprises an end (5) presenting fixing means (6) and counter-means (7) associated with a hollow tubular end (22) of the carrier (20) to which the catheter (2) of the first portion (100A) of the cannula needle (100) is secured.

4. A cannula needle as claimed in claim 3, **characterised in that** the fixing means are a pair of arched recesses or cuts (6) provided within an inner wall (50) of the end (5) of said first portion (31) of tubular element (30), said arched recesses or cuts (6) being arranged to removably receive portions (7) jutting, in opposing positions, from an edge (28) of said hollow tubular end (22), said portions (7) defining the fixing counter-means.

5. A cannula needle as claimed in claim 4, **characterised in that** at least one flattened portion (29) arranged to face the inner wall (50) of the end (5) of the tubular element (30) defining the protection member (3) is present between the portions (7) which jut from the edge of the hollow tubular end (22) of the first portion (100A) of the cannula needle (100).

6. A cannula needle as claimed in claims 3 and 4, **characterised in that** the end (5) of the tubular element (30) defining the protection member (3) presents an end recess (52) into which said arched recesses (6) open.

7. A cannula needle as claimed in claim 1, **characterised in that** the two portions (31, 34) of the tubular element (30) defining the protection member (3) each present a slot (36, 38), these slots defining the longitudinal aperture (11) within which the guide means (9) for the movement of the metal needle (1) slide.

8. A cannula needle as claimed in claim 7, **characterised in that** the two portions (31, 34) are hinged together via a weakening line (35), a substantially V-shaped recess being present between said two portions when these are spread out and lying adjacent and consecutive, to enable the metal needle (1) to move.

9. A cannula needle as claimed in claim 2, **characterised in that** the locking means (14) are a projection (14) provided in the interior of the protection member (3) to cooperate with a recess (13) associated with the support element (8) for the metal needle (1), said projection being positioned within said protection member (3) at a point such as to allow cooperation with the recess only when the metal needle (1) is completely inserted in the protection member (3).

10. A cannula needle as claimed in claim 9, **characterised in that** the locking means (14) are associated with the second portion of the tubular element (30) of the protection member (3).

11. A cannula needle as claimed in claim 10, **characterised in that** the locking means (14) are positioned in correspondence with an open end of the second portion (34) of the tubular element (30) of the protection member (3).

12. A cannula needle as claimed in claim 3, **characterised in that** the second portion (34) of the tubular element (30) defining the protection member (3) comprises external fins(61).

13. A cannula needle as claimed in claim 1, **characterised in that** the guide means for the metal needle (1) are a fin (9) rising from the support element (8) for said needle and rigid with this latter via a stiffening mount (91), said mount and said fin being slidable within the longitudinal aperture (11) of the protection member when the metal needle is separated from the catheter.

14. A cannula needle as claimed in claim 13, **characterised in that** at least part of the support element (8) is of transparent material.

15. A cannula needle as claimed in claim 14, **characterised in that** the transparent part of the support element (8) is the fin (9) defining the guide means.

16. A cannula needle as claimed in claim 13, **characterised in that** the support element presents, in proximity to the fin (9), a limit stop (88) for its connection to the hollow tubular end (22) of the carrier (20) of the first portion (100A) of the cannula (100).

17. A cannula needle as claimed in claim 1, **characterised by** being of the one-way type.

18. A cannula needle as claimed in claim 1, **characterised by** being of the two-way type.

19. A protection device for a metal needle (1) of a cannula needle (1(0), this latter comprising a first portion (100A) presenting a catheter (2) to be inserted into and maintained in a vein of a patient, and a second portion (100B) comprising the metal needle (1) to be inserted into the catheter (2) on inserting this latter into the vein, said metal needle (1) being associated with a support element (8), said catheter (2) being secured to a carrier (20) to be fixed to the patient when said catheter (2) is inserted into the patient's vein, said device being associable with said second portion (100B) of the cannula needle (100) and comprising tubular protection member (3) presenting a longitudinal aperture (11) within which there can slide guide means (9, 91) for the metal needle (1) associated with the second portion (100B) of said cannula needle (100) when the metal needle (1) is separated from the catheter (2), and arranged to completely receive the metal needle when this is separated from the catheter on separating the second portion (100B) of the cannula needle (100) from the first (100A) and the catheter remains in the vein, **characterised by** said tubular protection member (3) comprises a plurality of foldable portions (31, 34) arranged to be closable one over the other when said member (3) is associated with the carrier (20) and the metal needle (1) is inserted into the catheter (2) so as to reduce the protection member length, said portions (31, 34) being separated and put adjacent so as to receive the metal needle (1) when it is separated from said first portion (100A) of the cannula needle (100), the protection member (3) being defined by a tubular element (30) comprising two hinged-together superposable portions (31, 34), the first (31) of these portions (31, 34) being able to be removably coupled to the carrier (20) to which the catheter (2) is secured.

20. A protection device as claimed in claim 19, **characterised by** comprising, for locking the second portion (100B) of the cannula needle (100), means (14) associated with said tubular element (30) to block the movement of said second needle portion (100B) when the metal needle (1), separated from the catheter (2), is completely contained within the tubular element so as to be protected by this latter when said second needle portion (100B) is separated from the first needle portion (100A) with said tubular protection member (3).

## Patentansprüche

1. Kanülen-Nadel oder Katheter-Nadel (100) mit einem ersten Bereich (100A), der einen Katheter (2) aufweist, der in eine Vene von einem Patienten eingesetzt und darin gehalten wird, und mit einem zweiten Bereich (100B), der eine Metallnadel (1) aufweist, die in den Katheter (2) eingesetzt ist, wenn Letzterer in die Vene eingesetzt wird und dann davon getrennt wird, wobei die Metallnadel (1) mit einem Halteelement (8) in Beziehung steht, wobei der Katheter (2) an einem Träger (20) angebracht ist, der an dem Patienten befestigt wird, wenn der Katheter (2) in die Vene des Patienten eingesetzt ist, wobei ein rohrförmiges Schutzbauteil (3) mit dem Träger (20) des ersten Bereichs (100A) in Beziehung steht, um darin das Halteelement (8) für die Metallnadel (1) aufzunehmen, wenn Letztere sich noch in dem Katheter (2) befindet, wobei das rohrförmige Schutzbauteil (3) eine längsgerichtete Öffnung (11) aufweist, in der eine Führungseinrichtung (9) für die Metallnadel (1), die mit dem zweiten Bereich (100B) der Kanülen-Nadel (100) in Beziehung steht, verschoben werden kann, wenn die Metallnadel (1) von dem Katheter (2) getrennt wird, und wobei die Nadel (1) in dem rohrförmige Schutzbauteil (3) aufgenommen ist, wenn der zweite Bereich (100B) der Kanülen-Nadel (100) von dem ersten Bereich (100A) getrennt wird und der Katheter (2) in der Vene gehalten ist, **dadurch gekennzeichnet, dass** das rohrförmige Schutzbauteil (3) eine Vielzahl von knickbaren Bereichen (31, 34) aufweist, die dazu ausgestaltet sind, um übereinander verschließbar zu sein, wenn das Bauteil (3) mit dem Träger (20) in Beziehung steht und die Metallnadel (1) in den Katheter (2) eingesetzt ist, um so die Länge des Schutzbauteils zu reduzieren, wobei die Bereiche (31, 34) getrennt und benachbart zueinander angeordnet werden, um die Metallnadel (1) aufzunehmen, wenn diese von dem ersten Bereich (100A) der Kanülen-Nadel (100) getrennt wird, wobei das Schutzbauteil (3) durch ein rohrförmiges Element (30) gebildet ist, das zwei gelenkig verbundene Bereiche (31, 34) aufweist, die übereinander angeordnet werden können, und wobei der erste (31) dieser Bereiche (31, 34) dazu ausgestaltet ist, um abtrennbar mit dem Träger (20) verbunden zu werden, an dem der Katheter (2) angebracht ist.

2. Kanülen-Nadel nach Anspruch 1, **dadurch gekennzeichnet, dass** der zweite Bereich (100B) der Nadel Arretiereinrichtungen aufweist, die mit dem rohrförmigen Schutzbauteil (3) in Beziehung stehen, um eine Bewegung des zweiten Bereichs (100B) zu arretieren, wenn die Metallnadel (1), getrennt von dem Katheter (2), vollständig in dem rohrförmigen Schutzbauteil (3) aufgenommen ist, um so durch Letzteres geschützt zu sein, wenn der zweite Bereich (100B) der Nadel von dem ersten Bereich (100A) der Nadel mit dem rohrförmigen Schutzbauteil (3) getrennt ist.

3. Kanülen-Nadel nach Anspruch 1, **dadurch gekennzeichnet, dass** der erste Bereich (31) des rohrförmigen Elements (30), durch welches das Schutzbauteil (3) definiert ist, ein Ende (5) hat, das eine Befestigungseinrichtung (6) aufweist, und dass eine Gegeneinrichtung (7) mit einem hohlen rohrförmigen Ende (22) des Träger (20) in Beziehung steht, an dem der Katheter (2) des ersten Bereichs (100A) der Kanülen-Nadel (100) angebracht ist.

4. Kanülen-Nadel nach Anspruch 3, **dadurch gekennzeichnet, dass** die Befestigungseinrichtung ein Paar bogenförmige Aussparungen oder Einschnitte (6) umfasst, die in einer inneren Wand (50) von dem Ende (5) des ersten Bereichs (31) des rohrförmigen Elements (30) vorgesehen sind, wobei die bogenförmigen Aussparungen oder Einschnitte (6) ausgestaltet sind, um Bereiche (7) lösbar aufzunehmen, die in gegenüberliegenden Positionen von einer Kante (28) des hohlen rohrförmigen Endes (22) vorstehen, wobei durch diese Bereiche (7) die befestigende Gegeneinrichtung definiert ist.

5. Kanülen-Nadel nach Anspruch 4, **dadurch gekennzeichnet, dass** zumindest ein abgeflachter Bereich (29), der angeordnet ist, um der inneren Wand (50) von dem Ende (5) des rohrförmigen Elements (30) zugewandt zu sein, durch dass das Schutzbauteil (3) definiert ist, zwischen den Bereichen (7) vorgesehen ist, die von der Kante von dem hohlen rohrförmigen Ende (22) des ersten Bereichs (100A) der Kanülen-Nadel (100) vorstehen.

6. Kanülen-Nadel nach Ansprüchen 3 und 4, **dadurch gekennzeichnet, dass** das Ende (5) des rohrförmigen Elements (30), durch das das Schutzbauteil (3) definiert ist, eine Endaussparung (52) aufweist, in die sich die bogenförmigen Aussparungen (6) öffnen.

7. Kanülen-Nadel nach Anspruch 1, **dadurch gekennzeichnet, dass** die beiden Bereiche (31, 34) des rohrförmigen Elements (30), durch die das Schutzbauteil (3) definiert ist, jeweils einen Schlitz (36, 38) aufweist, wobei durch diese Schlitze die längsgerichtete Öffnung (11) gebildet ist, in der die Führungseinrichtung (9) für die Verlagerung der Metallnadel (1) verschoben werden kann.

8. Kanülen-Nadel nach Anspruch 7, **dadurch gekennzeichnet, dass** die beiden Bereiche (31, 34) entlang einer abgeschwächte Linie (35) gelenkig verbunden sind, wobei eine im wesentlichen V-förmige Aussparung zwischen diesen beiden Bereichen vorhanden ist, wenn diese auseinandergespreizt sind und benachbart und hintereinander angeordnet sind, um eine Verlagerung der Metallnadel (1) zu ermöglichen.

9. Kanülen-Nadel nach Anspruch 2, **dadurch gekennzeichnet, dass** die Arretiereinrichtungen (14) einen Vorsprung (14) beinhalten, der im Inneren des Schutzbauteils (3) ausgebildet ist, um mit einer Aussparung (13) zusammenzuwirken, die an dem Halteelement (8) für die Metallnadel (1) ausgebildet ist, wobei der Vorsprung in dem Schutzbauteil (3) an einer Stelle positioniert ist, um so ein Zusammenwirken mit der Aussparung nur dann zu ermöglichen, wenn die Metallnadel (1) vollständig in dem Schutzbauteil (3) aufgenommen ist.

10. Kanülen-Nadel nach Anspruch 9, **dadurch gekennzeichnet, dass** die Arretiereinrichtungen (14) mit dem zweiten Bereich des rohrförmigen Elements (30) des Schutzbauteils (3) in Beziehung stehen.

11. Kanülen-Nadel nach Anspruch 10, **dadurch gekennzeichnet, dass** die Arretiereinrichtungen (14) in Übereinstimmung mit einem offenen Ende des zweiten Bereichs (34) des rohrförmigen Elements (30) des Schutzbauteils (3) angeordnet sind.

12. Kanülen-Nadel nach Anspruch 3, **dadurch gekennzeichnet, dass** der zweite Bereich (34) des rohrförmigen Elements (30), durch das das Schutzbauteil (3) definiert ist, äußere Rippen (61) aufweist.

13. Kanülen-Nadel nach Anspruch 1, **dadurch gekennzeichnet, dass** die Führungseinrichtungen für die Metallnadel (1) eine Rippe (9) beinhalten, die von dem Halteelement (8) für die Nadel vorstehen und mit Letzterer über eine versteifende Halterung (91) starr verbunden ist, wobei die Halterung und die Rippe in der längsgerichteten Öffnung (11) des Schutzbauteils verschiebbar sind, wenn die Metallnadel von dem Katheter getrennt wird.

14. Kanülen-Nadel nach Anspruch 13, **dadurch gekennzeichnet, dass** zumindest ein Teil des Halteelements (8) aus einem transparenten Material hergestellt ist.

15. Kanülen-Nadel nach Anspruch 14, **dadurch gekennzeichnet, dass** das transparente Teil des Halteelements (8) die Rippe (9) ist, die zu den Führungseinrichtungen gehört.

16. Kanülen-Nadel nach Anspruch 13, **dadurch gekennzeichnet, dass** das Halteelement in der Nähe der Rippe (9) einen Begrenzungsanschlag (88) aufweist, und zwar für dessen Verbindung mit dem hohlen rohrförmigen Ende (22) des Trägers (20) des ersten Bereichs (100A) der Kanüle (100) .

17. Kanülen-Nadel nach Anspruch 1, **dadurch gekennzeichnet, dass** diese vom Einweg-Typ ist.

18. Kanülen-Nadel nach Anspruch 1, **dadurch gekennzeichnet, dass** diese vom Mehrweg-Typ ist.

19. Schutzvorrichtung für eine Metallnadel (1) von einer Kanülen-Nadel (100), wobei Letztere einen ersten Bereich (100A), der einen Katheter (2) aufweist, der in eine Vene von einem Patienten eingesetzt und darin gehalten wird, und einen zweite Bereich (100B) beinhaltet, der eine Metallnadel (1) aufweist, die in den Katheter (2) eingesetzt wird, wenn Letzterer in die Vene eingesetzt wird, wobei die Metallnadel (1) mit einem Halteelement (8) in Beziehung steht, wobei der Katheter (2) an einem Träger (20) angebracht ist, der an dem Patienten befestigt wird, wenn der Katheter (2) in die Vene des Patienten eingesetzt ist, wobei die Vorrichtung mit dem zweiten Bereich (100B) der Kanülen-Nadel (100) in Beziehung gebracht werden kann und ein rohrförmiges Schutzbauteil (3) aufweist, das eine längsgerichtete Öffnung (11) hat, in der eine Führungseinrichtung (9, 91) für die Metallnadel (1), die mit den zweiten Bereich (100B) der Kanülen-Nadel (100) in Beziehung steht, verschoben werden kann, wenn die Metallnadel (1) von dem Katheter (2) getrennt wird, und ausgestaltet ist, um die Metallnadel vollständig aufzunehmen, wenn diese von dem Katheter getrennt wird, wenn der zweite Bereich (100B) der Kanülen-Nadel (100) von dem ersten Bereich (100A) getrennt wird und der Katheter in der Vene verbleibt, **dadurch gekennzeichnet, dass** das rohrförmige Schutzbauteil (3) eine Vielzahl von knickbaren Bereichen (31, 34) aufweist, die dazu ausgestaltet sind, um übereinander verschließbar zu sein, wenn das Bauteil (3) mit dem Träger (20) in Beziehung steht und die Metallnadel (1) in den Katheter (2) eingesetzt ist, um so die Länge des Schutzbauteils zu reduzieren, wobei die Bereiche (31, 34) getrennt und benachbart zueinander angeordnet werden, um die Metallnadel (1) aufzunehmen, wenn diese von dem ersten Bereich (100A) der Kanülen-Nadel (100) getrennt wird, wobei das Schutzbauteil (3) durch ein rohrförmiges Element (30) gebildet ist, das zwei gelenkig verbundene Bereiche (31, 34) aufweist, die übereinander angeordnet werden können, und wobei der erste (31) dieser Bereiche (31, 34) dazu ausgestaltet ist, um abtrennbar mit dem Träger (20) verbunden zu werden, an dem der Katheter (2) angebracht ist.

20. Schutzvorrichtung nach Anspruch 19, **dadurch gekennzeichnet, dass** diese zum Arretieren des zweiten Bereichs (100B) der Kanülen-Nadel (100) Einrichtungen (14) aufweist, die mit dem rohrförmigen Element (30) in Beziehung stehen, um eine Bewegung des zweiten Bereichs (100B) der Nadel zu arretieren, wenn die Metallnadel (1), getrennt von dem Katheter (2), vollständig in dem rohrförmigen Element aufgenommen ist, um so durch Letzteres geschützt zu sein, wenn der zweite Bereich (100B) der Nadel von dem ersten Bereich (100A) der Nadel mit dem rohrförmigen Schutzbauteil (3) getrennt ist.

## Revendications

1. Aiguille canule ou aiguille cathéter (100) comprenant une première portion (100A) comportant un cathéter (2) à insérer et à maintenir dans une veine d'un patient, et une seconde portion (100B) comportant une aiguille métallique (1) insérée dans le cathéter (2) lors de l'insertion de cette dernière dans la veine et qui est ensuite séparée de celle-ci, ladite aiguille métallique (1) étant associée à un élément de support (8), ledit cathéter (2) étant fixé sur un support (20) à fixer sur le patient lorsque ledit cathéter (2) est introduit dans la veine du patient, un élément de protection tubulaire (3) étant associé au support (20) de la première portion (100A), de façon à y contenir l'élément de support (8) pour l'aiguille métallique (1) lorsque cette dernière est toujours à l'intérieur du cathéter (2), ledit élément de protection tubulaire (3) présentant une ouverture longitudinale (11) à l'intérieur de laquelle des moyens de guidage (9) pour l'aiguille métallique (1), associés à la seconde portion (100B) de ladite aiguille canule (100), peuvent coulisser lorsque l'aiguille métallique (1) est séparée du cathéter (2), ledit élément de protection tubulaire (3) contenant ladite aiguille métallique (1) lorsque la seconde portion (100B) de l'aiguille canule (100) est séparée de la première (100A) et le cathéter (2) est maintenu à l'intérieur de la veine, **caractérisée en ce que** l'élément de protection tubulaire (3) comprend une pluralité de portions pliables (31, 34) disposées de façon à pouvoir se fermer l'une sur l'autre lorsque ledit élément (3) est associé au support (20) et l'aiguille métallique (1) est insérée dans le cathéter (2), de façon à réduire la longueur de l'élément de protection, lesdites portions (31, 34) étant séparées et étant placées de façon contiguë afin de recevoir l'aiguille métallique (1) lorsqu'elle est détachée de ladite première portion (100A) de l'aiguille canule (100), ledit élément de protection (3) étant défini par un élément tubulaire (30) comprenant deux portions superposables articulées ensemble (31, 34), la première (31) de ces portions (31, 34) pouvant être accouplée de façon amovible au support (20) sur lequel est fixé le cathéter (2).

2. Aiguille canule selon la revendication 1, **caractérisée par** le fait de comprendre la seconde portion d'aiguille (100B) qui présente des moyens de verrouillage associés audit élément de protection tubulaire (3) pour verrouiller le mouvement de ladite seconde portion (100B) lorsque l'aiguille métallique (1), séparée du cathéter (2), est entièrement contenue à l'intérieur de l'élément de protection tubulaire (3), de façon à être protégée par ce dernier lorsque ladite seconde portion d'aiguille (100B) est séparée de la première portion d'aiguille (100A) avec ledit élément de protection tubulaire.

3. Aiguille canule selon la revendication 1, **caractérisée en ce que** ladite première portion (31) de l'élément tubulaire (30) définissant l'élément de protection (3) comprend une extrémité (5) présentant des moyens de fixation (6) et des contre -moyens (7) associés à une extrémité tubulaire creuse (22) du support (20) sur lequel est fixé le cathéter (2) de la première portion (100A) de l'aiguille canule (100).

4. Aiguille canule selon la revendication 3, **caractérisée en ce que** les moyens de fixation consistent en une paire d'évidements arqués ou de découpes (6) disposés à l'intérieur d'une paroi interne (50) de l'extrémité (5) de ladite première portion (31) de l'élément tubulaire (30), lesdits évidements arqués ou découpes (6) étant disposés de façon à recevoir de façon amovible des portions (7) en saillie dans des positions en regard, à partir d'un bord (28) de ladite extrémité tubulaire creuse (22), lesdites portions (7) définissant les contre- moyens de fixation.

5. Aiguille canule selon la revendication 4, **caractérisée en ce qu'**au moins une portion aplatie (29) disposée en regard de la paroi interne (50) de l'extrémité (5) de l'élément tubulaire (30) définissant l'élément de protection (3) est présente entre les portions (7) qui font saillie à partir du bord de l'extrémité tubulaire creuse (22) de la première portion (100A) de l'aiguille canule (100).

6. Aiguille canule selon la revendication 3 et 4, **caractérisée en ce que** l'extrémité (5) de l'élément tubulaire (30) définissant l'élément de protection (3) présente un évidement d'extrémité (52) dans lequel s'ouvrent lesdits évidements arqués (6).

7. Aiguille canule selon la revendication 1, **caractérisée en ce que** les deux portions (31, 34) de l'élément tubulaire (30) définissant l'élément de protection (3) présentent chacune une fente (36, 38), ces fentes définissant l'ouverture longitudinale (11) à l'intérieur de laquelle coulissent les moyens de guidage (9) pour le mouvement du coulisseau de l'aiguille métallique (1).

8. Aiguille canule selon la revendication 7, **caractérisée en ce que** les deux portions (31, 34) sont articulées ensemble par le biais d'une ligne de faiblesse (35), un évidement sensiblement en forme de V étant présent entre lesdites deux portions lorsque celles-ci sont écartées et se situent de façon contiguë et consécutive, pour permettre le déplacement de l'aiguille métallique (1).

9. Aiguille canule selon la revendication 2, **caractérisée en ce que** les moyens de verrouillage (14) sont une projection (14) disposée à l'intérieur de l'élément de protection (3) pour coopérer avec un évidement (13) associé à l'élément de support (8) pour l'aiguille métallique (1), ladite saillie étant positionnée à l'intérieur dudit élément de protection (3) en un point tel à permettre la coopération avec l'évidement uniquement lorsque l'aiguille métallique (1) est entièrement insérée dans l'élément de protection (3).

10. Aiguille canule selon la revendication 9, **caractérisée en ce que** les moyens de verrouillage (14) sont associés à la seconde portion de l'élément tubulaire (30) de l'élément de protection (3).

11. Aiguille canule selon la revendication 10, **caractérisée en ce que** les moyens de verrouillage (14) sont positionnés en correspondance avec une extrémité ouverte de la seconde portion (34) de l'élément tubulaire (30) de l'élément de protection (3).

12. Aiguille canule selon la revendication 3, **caractérisée en ce que** la seconde portion (34) de l'élément tubulaire (30) définissant l'élément de protection (3) comprend des ailettes externes (61).

13. Aiguille canule selon la revendication 1, **caractérisée en ce que** les moyens de guidage pour l'aiguille métallique (1) consistent en une ailette (9) s'élevant à partir de l'élément de support (8) pour ladite aiguille et rigide avec cette dernière au moyen d'un montage de rigidification (91), ledit montage et ladite ailette pouvant coulisser à l'intérieur de l'ouverture longitudinale (11) de l'élément de protection lorsque l'aiguille métallique est séparée du cathéter.

14. Aiguille canule selon la revendication 13, **caractérisée en ce qu'**au moins une partie de l'élément de support (8) est en matériau transparent.

15. Aiguille canule selon la revendication 14, **caractérisée en ce que** la partie transparente de l'élément de support (8) est l'ailette (9) définissant les moyens de guidage.

16. Aiguille canule selon la revendication 13, **caractérisée en ce que** l'élément de support présente, à proximité de l'ailette (9), une butée de fin de course (88) pour son raccordement sur l'extrémité tubulaire creuse (22) du support (20) de la première portion (100A) de la canule (100).

17. Aiguille canule selon la revendication 1, **caractérisée par** le fait d'être du type unidirectionnel.

18. Aiguille canule selon la revendication 1, **caractérisée par** le fait d'être du type bidirectionnel.

19. Dispositif de protection pour une aiguille métallique (1) d'une aiguille canule (100), cette dernière comprenant une première portion (100A) présentant un cathéter (2) à insérer et à maintenir dans une veine d'un patient, et une seconde portion (100B) comprenant l'aiguille métallique (1) à insérer dans le cathéter (2) lors de l'introduction de ce dernier dans la veine, ladite aiguille métallique (1) étant associée à un élément de support (8), ledit cathéter (2) étant fixé sur un support (20) à fixer sur le patient lorsque ledit cathéter (2) est introduit dans la veine du patient, ledit dispositif pouvant être associé à la seconde portion (100B) de l'aiguille canule (100), et comprenant un élément de protection tubulaire (3) qui présente une ouverture longitudinale (11) à l'intérieur de laquelle les moyens de guidage (9, 91) peuvent coulisser pour l'aiguille métallique (1) associée à la seconde portion (100B) de ladite aiguille canule (100) lorsque l'aiguille métallique (1) est séparée du cathéter (2), et adapté pour recevoir complètement l'aiguille métallique quand elle est séparée du cathéter lorsque la seconde portion (100B) de l'aiguille canule (100) est séparée de la première portion (100A) et le cathéter est maintenu à l'intérieur de la veine, **caractérisé par le fait que** ledit élément de protection tubulaire (3) comprend une pluralité de portions pliables (31, 34), de façon à pouvoir se fermer l'une sur l'autre lorsque ledit élément (3) est associé au support (20) et l'aiguille métallique (1) est introduite dans le cathéter (2) de façon à réduire la longueur de l'élément de protection, lesdites portions (31, 34) étant séparées et placées de façon contiguë afin de recevoir l'aiguille métallique (1) lorsqu'elle est séparée de ladite première portion (100A) de l'aiguille canule (100), l'élément de protection (3) étant défini par un élément tubulaire (30) comprenant deux portions superposables articulées ensemble (31, 34), la première (31) de ces portions (31, 34) pouvant être accouplée de façon amovible sur le support (20) sur lequel est fixé le cathéter (2).

20. Dispositif de protection selon la revendication 19, **caractérisé par** le fait de comprendre, pour le verrouillage de la seconde portion (100B) de l'aiguille canule 100, des moyens (14) associés audit élément tubulaire (30) pour verrouiller le mouvement de ladite seconde portion d'aiguille (100B) lorsque l'aiguille métallique (1), séparée du cathéter (2), est entièrement contenue à l'intérieur de l'élément tubulaire, de façon à être protégée par ce dernier lorsque ladite seconde portion d'aiguille (100B) est séparée de la première portion (100A) avec ledit élément de protection tubulaire de portion d'aiguille (3).
